# EUROPÄISCHE PATENTSCHRIFT

(11) **EP 3 426 168 B1**
(45) Veröffentlichungstag und Bekanntmachung des Hinweises auf die Patenterteilung: **01.04.2020**
(21) Anmeldenummer: 17707513.2
(22) Anmeldetag: 23.02.2017
(51) Int. Cl.: A61B 17/221, A61B 17/29, A61B 17/22, A61B 17/00

(54) **HANDBETÄTIGTES FUNKTIONSSCHLAUCHINSTRUMENT**
HAND-OPERATED FUNCTIONAL HOSE INSTRUMENT
INSTRUMENT À TUYAU SOUPLE FONCTIONNEL À COMMANDE MANUELLE

(30) Priorität: 11.03.2016 DE 102016204092
(43) Veröffentlichungstag der Anmeldung: 16.01.2019
(73) Patentinhaber: EPflex Feinwerktechnik GmbH, 72581 Dettingen/Erms (DE)
(72) Erfinder: UIHLEIN, Bernhard, 72581 Dettingen (DE)
(74) Vertreter: Patentanwälte Ruff, Wilhelm, Beier, Dauster & Partner mbB
(86) Internationale Anmeldenummer: PCT/EP2017/054217
(87) Internationale Veröffentlichungsnummer: WO 2017/153178

(56) Entgegenhaltungen:
- WO-A1-2010/133245
- WO-A1-2011/095233
- US-A1- 2003 009 176
- US-A1- 2005 240 120
- US-B1- 6 551 327

## Beschreibung

Die Erfindung bezieht sich auf ein handbetätigtes Funktionsschlauchinstrument nach dem Oberbegriff des Anspruchs 1.

Ein solches Instrument umfasst ein schlauchförmiges Funktionsteil und ein sich in diesem erstreckendes drahtförmiges Funktionsteil, wobei die beiden Funktionsteile axial relativbeweglich sind, um an einem distalen Ende eine Nutzfunktion auszuüben. Weiter beinhaltet das Instrument eine zum lösbaren Koppeln mit einem Bediengriffkörper eingerichtete Betätigungseinheit, die an einem proximalen Endabschnitt der Funktionsteile angeordnet ist und ein erstes und zweites Betätigungsteil umfasst, die axial relativbeweglich miteinander gekoppelt sind, wobei eines der beiden Betätigungsteile eine Schlauchfixierung aufweist, durch die das schlauchförmige Funktionsteil an ihm fixiert ist, und das andere der beiden Betätigungsteile eine Drahtfixierung aufweist, durch die das drahtförmige Funktionsteil an ihm fixiert ist. Das erste Betätigungsteil beinhaltet ein Axialfixierelement zum axialen Fixieren des ersten Betätigungsteils am Bediengriffkörper, und das zweite Betätigungsteil weist ein fingerbetätigtes Bedienschieberelement auf. Für die Schlauchfixierung und die Drahtfixierung sind beliebige Fixierungen herkömmlicher Art verwendbar, wie Verkleben, Verschweißen sowie lösbare und unlösbare formschlüssige oder kraftschlüssige Verbindungen.

Unter dem Begriff schlauchförmiges Funktionsteil ist dabei vorliegend allgemein ein beliebiges langgestrecktes Bauteil zu verstehen, das einen Hohlkanal zur Durchführung des drahtförmigen Funktionsteils aufweist, wobei es sich je nach Anwendungsfall um ein gegenüber dem drahtförmigen Funktionsteil biegeweicheres oder biegesteiferes Schlauch- bzw. Rohrteil handeln kann. Unter dem Begriff drahtförmiges Funktionsteil ist vorliegend ein beliebiges langgestrecktes, im Hohlkanal des schlauchförmigen Funktionsteils axial relativbeweglich aufgenommenes, drahtförmiges Bauteil aus einem Metall- oder Kunststoffmaterial zu verstehen.

Handbetätigte Funktionsschlauchinstrumente dieser und anderer Art sind beispielsweise in der endoskopischen Medizintechnik gebräuchlich, speziell in Form von Steinfangkorbinstrumenten mit entfaltbarem Drahtkorb zum Einfangen von Steinen und dergleichen in Gewebehohlräumen sowie ähnlichen Instrumenten, wie Drahtfilterinstrumenten, Drahtschlingeninstrumenten und Fangnetzinstrumenten. Bei diesen genannten Anwendungen befindet sich am distalen Ende des drahtförmigen Funktionsteils ein typischerweise entfaltbares Element, wie ein Drahtkorb, ein Drahtfilter, eine Drahtschlinge oder ein Fangnetz, und durch axiales Zurück- und Vorbewegen des drahtförmigen Funktionsteils relativ zum dieses umgebenden schlauchförmigen Funktionsteil wird dieses Element in das distale Ende des schlauchförmigen Funktionsteils unter Zusammenfalten eingezogen bzw. unter Entfalten herausbewegt. Eine ähnliche Anwendung sind Führungsdrahteinheiten für Katheterinstrumente, wobei es sich in diesem Fall beim drahtförmigen Funktionsteil um einen sogenannten Zugdraht und beim schlauchförmigen Funktionsteil um einen diesen umgebenden Schlauch handelt, der im distalen Bereich mit dem Zugdraht verbunden ist. Durch axiale Relativbewegung von Zugdraht und Schlauch kann ein distaler Abschnitt der Führungsdrahteinheit in einer gewünschten Weise verformt, z.B. gebogen werden, siehe dazu beispielsweise die Offenlegungsschrift US 2005/0113862 A1.

Die axiale Relativbewegung wird durch Bedienung der Betätigungseinheit am proximalen Endabschnitt des Funktionsschlauchinstruments bewirkt. Für die Bedienung ist das Funktionsschlauchinstrument mit seiner proximalen Betätigungseinheit typischerweise an einen Bediengriff gekoppelt. Herkömmliche Auslegungen der Betätigungseinheit und eines zugehörigen Bediengriffs sind meist relativ komplex in ihrem Aufbau und/oder ihrer Kopplung, und häufig ist der Bediengriff unlösbar mit der Betätigungseinheit bzw. den beiden Funktionsteilen verbunden. Um diesbezüglich eine Verbesserung zu erreichen, wird in der Offenlegungsschrift WO 2011/095233 A1 ein handbetätigtes Funktionsschlauchinstrument der eingangs genannten, gattungsgemäßen Art vorgeschlagen.

Die Offenlegungsschrift DE 10 2010 037 618 A1 offenbart eine medizinische Vorrichtung mit einem flexiblen Katheter, an dessen proximales Ende eine Betätigungseinheit anschließt, während er am distalen Ende ein Arbeitselement aufweist, das durch ein Zug- oder Druckelement betätigt wird, welches drehbar durch den Katheter hindurch geführt ist. Die Betätigungseinheit kann z.B. ein Röhrchen mit eingeformtem Langloch und eine Führungshülse beinhalten, die in dem Röhrchen gleiten kann und an welcher das Zug-/Druckelement in Form eines Drahtes proximal fixiert ist. Ein Ringgriff ist auf eine Mittelhülse aufgeschoben und mit ihr so verbunden, dass sie gegenüber dieser drehbar, jedoch nicht axial verschiebbar ist, wobei die Mittelhülse ihrerseits auf das Röhrchen aufgeschoben und mit der Führungshülse verbunden ist. Am proximalen Ende des Röhrchens ist spannzangenartig ein ösenförmiger Griff drehbeweglich axial fixiert. Zum axialen Verschieben des Drahtes werden der Ringgriff und die Mittelhülse auf dem Röhrchen axial verschoben. Zum Drehen des Drahtes dient eine vorgelagert angeordnete Drehhülse, die mit ihrem proximalen Ende auf ein distales Gewinde des Röhrchens aufgeschraubt ist und die der Benutzer mit einer Hand drehen kann, während er mit der anderen Hand den Ringgriff und den ösenförmigen Griff ortsfest hält.

Die Offenlegungsschrift US 2012/0095477 A1 offenbart ein medizinisches Steinfangkorbinstrument mit einem Bediengriff, an welchem ein daumenbetätigbarer Schieber axialbeweglich gehalten ist, um das Fangkörbchen aus einem Schlauch oder Rohr auszufahren bzw. wieder einzuziehen. Zusätzlich ist am proximalen Ende des Griffs ein Drehknopf angeordnet, mit dem der Benutzer, wenn er das Instrument mit der einen Hand am Bediengriff hält, das Fangkörbchen drehen kann, indem er mit der anderen Hand den Drehknopf betätigt.

Die Patentschrift US 5,219,332 A offenbart ein an einer beliebigen gewünschten Stelle auf einen medizinischen Führungsdraht aufsetzbares Bedienhilfselement, durch das der Führungsdraht für Längs- oder Drehbetätigungen besser gegriffen werden kann. Das Bedienhilfselement beinhaltet zwei korrespondierende, axial zusammensteckbare Rohrteile und ein darin aufnehmbares Arretierteil aus Elastomermaterial. Die Rohrteile weisen jeweils einen über ihre Länge durchgehenden Axialschlitz auf, durch den hindurch der Führungsdraht eingeführt werden kann. Durch relatives Verdrehen der beiden Rohrteile fixiert das Arretierteil den Führungsdraht gegenüber den Rohrteilen, indem sich das Arretierteil verformend zwischen dem Führungsdraht und der Innenwand der Rohrteile verspannt.

Der Erfindung liegt als technisches Problem die Bereitstellung eines handbetätigten Funktionsschlauchinstruments der eingangs genannten Art zugrunde, das gegenüber dem oben erwähnten Stand der Technik weiter verbessert ist und insbesondere hinsichtlich seiner Funktionalität und/oder seines Aufbaus.

Die Erfindung löst dieses Problem durch die Bereitstellung eines handbetätigten Funktionsschlauchinstruments mit den Merkmalen des Anspruchs 1. Vorteilhafte Weiterbildungen der Erfindung sind in den Unteransprüchen angegeben, deren Wortlaut hiermit durch Verweis in die Beschreibung aufgenommen wird.

Erfindungsgemäß umfasst die Drahtfixierung zwei gegeneinander um eine Längsachse von einer Drahtfreigabestellung in eine Drahtklemmstellung verdrehbare Drahtfixierteile mit je einer exzentrischen axialen Drahtdurchführungsöffnung, wobei die beiden Durchführungsöffnungen axial hintereinander angeordnet sind und in der Drahtfreigabestellung fluchten und in der Drahtklemmstellung außer Flucht sind. Diese Drahtfixierung ist fertigungstechnisch einfach realisierbar und stellt funktionssicher eine ausreichende Klemmkraft für das drahtförmige Funktionsteil bereit. Zur Fixierung am entsprechenden Betätigungsteil wird das drahtförmige Funktionsteil mit seinem proximalen Endabschnitt durch die fluchtenden Durchführungsöffnungen in der Drahtfreigabestellung der Drahtfixierteile hindurchgesteckt, wonach die Drahtfixierteile in ihre Drahtklemmstellung verdreht werden. Die exzentrischen Durchführungsöffnungen gelangen dadurch außer Flucht, was eine Verbiegung und Verformung des hindurchgesteckten Abschnitts des drahtförmigen Funktionsteils und auf diese Weise die gewünschte festgeklemmte Fixierung des drahtförmigen Funktionsteils am betreffenden Betätigungsteil zur Folge hat.

Bei Bedarf lassen sich alle Komponenten der Betätigungsmimik des erfindungsgemäßen Funktionsschlauchinstruments als Kunststoffteile in Spritzgusstechnik herstellen, und die Montage der Bedieneinheit mit den beiden Funktionsteilen an einem zugehörigen Bediengriff lässt sich rasch und unkompliziert und vorzugsweise ohne Werkzeug durch eine lösbare Kopplung bewerkstelligen. Unter lösbarer Kopplung ist hierbei eine zerstörungsfrei lösbare Verbindung zu verstehen, die bei Bedarf wiederholt hergestellt und wieder gelöst werden kann. Es ist zudem eine Herstellung vollständig ohne metallische Komponenten möglich, was für medizinische Endoskopieanwendungen nützlich sein kann, bei denen Magnetresonanztechnik eingesetzt wird. Der separate Bediengriff kann unabhängig von der Bedieneinheit und den beiden schlauch- bzw. drahtförmigen Funktionsteilen gefertigt und in seiner Gestaltung z.B. unter ergonomischen und handhabungstechnischen Gesichtspunkten optimiert werden.

In einer Weiterbildung der Erfindung weist das erste Betätigungsteil ein fingerbetätigtes Bediendrehelement auf. Dies erlaubt es einem Benutzer bequem, das erste Betätigungsteil und damit auch das an ihm fixierte schlauch- oder drahtförmige Funktionsteil relativ zum Bediengriffkörper zu drehen, wozu das erste Betätigungsteil gegenüber dem Bediengriffkörper drehbeweglich angeordnet ist, was eine entsprechende drehbewegliche Realisierung seines Axialfixierelements umfasst. Das Instrument ist in einer vorteilhaften Realisierung für eine Einhandbedienbarkeit ausgelegt, d.h. es kann in diesem Fall mit nur einer Hand bedient werden.

In einer Weiterbildung der Erfindung weist das erste Betätigungsteil einen distalen Konusabschnitt mit einer axialen Ausnehmung auf, in die das zweite Betätigungsteil wenigstens partiell einschiebbar ist. Dies ermöglicht bei Bedarf eine vergleichsweise kurze Bauform für die Betätigungseinheit und damit auch für den Bediengriff. Außerdem kann diese Maßnahme die axialbewegliche Führung der beiden Betätigungsteile aneinander unterstützen.

In einer Weiterbildung der Erfindung weist das zweite Betätigungsteil einen mit dem ersten Betätigungsteil drehfest und axialbeweglich gekoppelten Fixierkörper auf, mit dem das Bedienschieberelement drehbeweglich und axial fixiert gekoppelt ist. Durch diese vorteilhafte Konstruktion wird, wenn der Benutzer über das Bediendrehelement das erste Betätigungsteil dreht, der Fixierkörper des ersten Betätigungsteils synchron mitgedreht, während das Bedienschieberelement nicht mitgedreht wird und daher seine Lage relativ zum Bediengriff in der Drehrichtung nicht ändert. Der Fixierkörper des ersten Betätigungsteils dient zur Fixierung des schlauchförmigen oder des drahtförmigen Funktionsteils, während das andere Funktionsteil am zweiten Betätigungsteil fixiert ist. Dies hat den vorteilhaften Effekt, dass sich bei der über das Bediendrehelement vom Benutzer initiierten Drehbewegung das schlauchförmige und das drahtförmige Funktionsteil synchron drehen. Das vermeidet etwaige Reibungsverluste, die durch eine Relativdrehung der beiden Funktionsteile entstehen könnten.

In einer Weiterbildung der Erfindung weist eines der beiden Betätigungsteile eine Führungshülse auf, während das andere Betätigungsteil ein in dieser Führungshülse drehfest und axial beweglich geführtes Führungselement beinhaltet. Dies trägt zu einer sicheren Führung der beiden relativ zueinander axialbeweglichen Betätigungsteile aneinander bei.

In einer Ausgestaltung dieser Maßnahme weisen die beiden Betätigungsteile Endanschläge zur beidseitigen Begrenzung der axialen Relativbewegung von Führungshülse und Führungselement auf. Dadurch bleiben die beiden axial relativbeweglichen Betätigungsteile gesichert auch dann miteinander verbunden, wenn sie noch nicht am Bediengriff montiert sind oder wenn sie von diesem abgenommen worden sind.

In einer Weiterbildung der Erfindung sind die beiden Drahtfixierteile durch einen rohrförmigen Fixierendabschnitt des betreffenden Betätigungsteils und einen an diesen axial ansteckbaren Fixierstutzen gebildet. Dies ist fertigungstechnisch einfach realisierbar und erweist sich funktionstechnisch vorteilhaft und sicher. Der Fixierstutzen braucht nur axial auf oder in den Fixierendabschnitt gesteckt werden. In der gesteckten Lage braucht er dann zum Festklemmen oder Lösen des drahtförmigen Funktionsteils lediglich entsprechend gegenüber dem Fixierstutzen verdreht werden.

In einer Weiterbildung der Erfindung weist das erste Betätigungsteil die Schlauchfixierung auf, während das zweite Betätigungsteil die Drahtfixierung beinhaltet. In einer vorteilhaften Ausführung bewirkt der Benutzer die axiale Relativbewegung der beiden Betätigungsteile durch axiales Verschieben des Bedienschieberelements relativ zum Bediengriff. Dies bedeutet dann, dass das drahtförmige Funktionsteil aktiv axial gegenüber dem schlauchförmigen Funktionsteil vor und zurück bewegt werden kann. Bei einem medizinischen Steinfangkorbinstrument kann auf diese Weise das distal am drahtförmigen Funktionsteil vorgesehene Fangkörbchen aus dem umgebenden schlauchförmigen Funktionsteil nach vorn herausbewegt und entfaltet bzw. wieder in selbiges zurückgezogen werden.

In einer alternativen Weiterbildung weist das erste Betätigungsteil die Drahtfixierung auf, während das zweite Betätigungsteil die Schlauchfixierung beinhaltet. In diesem Fall kann dann der Benutzer z.B. durch aktives axiales Verschieben des Bedienschieberelements relativ zum Bediengriff das schlauchförmige Funktionsteil relativ zum drahtförmigen Funktionsteil vorschieben oder zurückschieben. Im Fall des erwähnten Steinfangkorbinstruments lässt sich auf diese Weise das Fangkörbchen durch aktives Zurückziehen des schlauchförmigen Funktionsteils entfalten und durch Vorschieben des schlauchförmigen Funktionsteils wieder in das schlauchförmige Funktionsteil zusammenfalten, wobei das drahtförmige Funktionsteil und damit auch das Fangkörbchen seine axiale Lage beibehält. Dies kann für den Vorgang zum Einfangen von Nierensteinen und anderen aus einem tierischen oder menschlichen Organismus zu entfernenden Partikeln vorteilhaft sein.

In einer Weiterbildung der Erfindung ist das Bediendrehelement distal vor dem Bedienschieberelement angeordnet. Dies erleichtert eine komfortable Bedienung des Instruments durch den Benutzer z.B. mit nur einer Hand, wobei er z.B. das Bedienschieberelement mit dem Daumen und das Bediendrehelement auch mit dem Daumen oder mit Daumen und Zeigefinger betätigen kann.

In einer alternativen Weiterbildung ist das Bediendrehelement proximal hinter dem Bedienschieberelement angeordnet. Auch diese Realisierung ermöglicht eine Einhandbedienung des Instruments. Dazu greift der Benutzer den Bediengriff in entgegengesetzter Handposition, d.h. mit der Daumenseite seiner den Griff erfassenden Hand in proximaler statt distaler Instrumentenrichtung.

In einer Weiterbildung der Erfindung umfasst das Funktionsschlauchelement einen Bediengriff mit einem Griffkörper, der zum lösbaren Aufnehmen der Betätigungseinheit eingerichtet ist und ein Koppelelement zum axial fixierten Ankoppeln des ersten Betätigungsteils aufweist. Das lösbare Aufnehmen der Betätigungseinheit erleichtert die Montage derselben am Bediengriff und ermöglicht bei Bedarf ein einfaches Abnehmen der Betätigungseinheit vom Bediengriff und erneutes Wiederanbringen desselben oder einer anderen, insoweit baugleichen Betätigungseinheit. Durch das axial fixierte Ankoppeln des ersten Betätigungsteil am Bediengriffkörper werden bei Betätigung des am zweiten Betätigungsteil angeordneten Bedienschieberelements das zweite Betätigungsteil und das an ihm fixierte Funktionsteil aktiv relativ zum Bediengriffkörper nach vorn oder hinten bewegt, während das erste Betätigungsteil und das daran fixierte Funktionsteil ihrer axiale Lage relativ zum Bediengriffkörper beibehalten.

In einer Ausgestaltung dieser Maßnahme ist das Koppelelement des Griffkörpers zum drehbeweglichen Ankoppeln des ersten Betätigungsteils eingerichtet. Bei Betätigung des Bediendrehelements lässt sich dann das erste Betätigungsteil, vorzugsweise zusammen mit dem zweiten Betätigungsteil bzw. einem Fixierkörper desselben, relativ zum Bediengriffkörper drehen.

In einer anderweitigen Ausgestaltung weist der Griffkörper eine Axialführung für das Bedienschieberelement auf. Dies kann eine sichere Führung der Axialbewegung des Bedienschieberelements am Bediengriffkörper unterstützen.

In einer anderweitigen Ausgestaltung ist das Bediendrehelement an einer distalen oder proximalen Stirnseite des Griffkörpers angeordnet. Diese Positionen für das Bediendrehelement eignen sich besonders gut für eine Einhandbedienung des Instruments.

In einer weiteren Ausgestaltung ist der Griffkörper mehrteilig ausgeführt, wobei er ein Griffbasisteil und ein mit diesem lösbar verbindbares Griffkoppelteil umfasst und wobei das Griffkoppelteil zum lösbaren Aufnehmen der Betätigungseinheit eingerichtet ist und das Koppelelement zum Ankoppeln des ersten Betätigungsteils aufweist. Diese Ausgestaltung lässt eine vergleichsweise einfache Montage des Instruments zu und bietet fertigungstechnische Vorteile.

In einer Weiterbildung der Erfindung ist wenigstens eine der beiden Drahtdurchführungsöffnungen eine umfangsseitig geschlossene Öffnung. Dies kann zu einer sehr guten Klemm- bzw. Fixierwirkung der drahtklemmend zusammenwirkenden Drahtfixierteile beitragen.

Vorteilhafte Ausführungsformen der Erfindung sind in den Zeichnungen dargestellt und werden nachfolgend beschrieben. Hierbei zeigen:
- Fig. 1: eine Perspektivansicht eines als medizinisches Steinfangkorbinstrument verwendbaren handbetätigten Funktionsschlauchinstruments,
- Fig. 2: eine perspektivische Explosionsansicht eines proximalen, bedienseitigen Teils des Instruments von Fig. 1,
- Fig. 3: eine perspektivische Explosionsansicht einer Betätigungseinheit und eines Bediengriffs des Instruments Fig. 1,
- Fig. 4: eine Längsschnittansicht des Instrumententeils von Fig. 2 im montierten Zustand von Fig. 1,
- Fig. 5: eine teillängsgeschnittene Seitenansicht des Instrumententeils von Fig. 2 im montierten Zustand entsprechend Fig. 4,
- Fig. 6: eine Längsschnittansicht der Betätigungseinheit des Instruments von Fig. 1 mit zwei axialbeweglich gekoppelten Betätigungsteilen,
- Fig. 7: eine Längsschnittansicht eines ersten der beiden Betätigungsteile von Fig. 6 mit daran fixiertem schlauchförmigem Funktionsteil und hindurchgeführtem drahtförmigem Funktionsteil,
- Fig. 8: eine Längsschnittansicht nur des Betätigungsteils von Fig. 7 in einer zu derjenigen von Fig. 7 senkrechten Schnittebene,
- Fig. 9: eine perspektivische Explosionsansicht zweier Komponenten eines zweiten der beiden Betätigungsteile von Fig. 6,
- Fig. 10: eine Perspektivansicht eines hülsenförmigen Drahtfixierkörpers von Fig. 9,
- Fig. 11: eine Längsschnittansicht durch den Drahtfixierkörper von Fig. 10,
- Fig. 12: eine Querschnittansicht längs einer Linie XII-XII von Fig. 6,
- Fig. 13: eine Längsschnittansicht der beiden Komponenten von Fig. 9 in montiertem Zustand in einer Drahtfreigabestellung,
- Fig. 14: eine Längsschnittansicht entsprechend Fig. 13 in einer Drahtklemmstellung,
- Fig. 15: eine Querschnittansicht entsprechend Fig. 12 für eine Ausführungsvariante,
- Fig. 16: eine Querschnittansicht entsprechend Fig. 12 für eine weitere Ausführungsvariante,
- Fig. 17: eine Explosionsansicht entsprechend Fig. 9 für eine Ausführungsvariante,
- Fig. 18: eine Längsschnittansicht entsprechend Fig. 14 für die Ausführungsvariante von Fig. 17,
- Fig. 19: eine Explosionsansicht entsprechend Fig. 9 für eine weitere Ausführungsvariante,
- Fig. 20: eine Längsschnittansicht entsprechend Fig. 14 für die Ausführungsvariante von Fig. 19,
- Fig. 21: eine Seitenansicht einer weiteren Ausführungsvariante für eine der beiden Komponenten von Fig. 9,
- Fig. 22: eine Perspektivansicht eines zur Komponente von Fig. 21 passenden Klemmstifts als Alternative zum hülsenförmigen Fixierkörper von Fig. 10,
- Fig. 23: eine Seitenansicht des Instruments von Fig. 1 in einer Gebrauchshaltung durch einen Nutzer,
- Fig. 24: eine Explosionsansicht entsprechend Fig. 2 für eine Ausführungsvariante,
- Fig. 25: eine Explosionsansicht entsprechend Fig. 3 für die Ausführungsvariante von Fig. 24,
- Fig. 26: eine Längsschnittansicht entsprechend Fig. 6 für die Ausführungsvariante von Fig. 24,
- Fig. 27: eine Perspektivansicht entsprechend Fig. 1 für eine weitere Ausführungsvariante,
- Fig. 28: eine Explosionsansicht entsprechend Fig. 3 für die Ausführungsvariante von Fig. 27,
- Fig. 29: eine Längsschnittansicht entsprechend Fig. 4 für die Ausführungsvariante von Fig. 27,
- Fig. 30: eine Ansicht entsprechend Fig. 23 für die Ausführungsvariante von Fig. 27,
- Fig. 31: eine Perspektivansicht entsprechend Fig. 1 für eine weitere Ausführungsvariante,
- Fig. 32: eine Explosionsansicht entsprechend Fig. 3 für die Ausführungsvariante von Fig. 31,
- Fig. 33: eine Längsschnittansicht entsprechend Fig. 4 für die Ausführungsvariante von Fig. 31 und
- Fig. 34: eine perspektivische Explosionsansicht entsprechend Fig. 32 für eine weitere Ausführungsvariante.

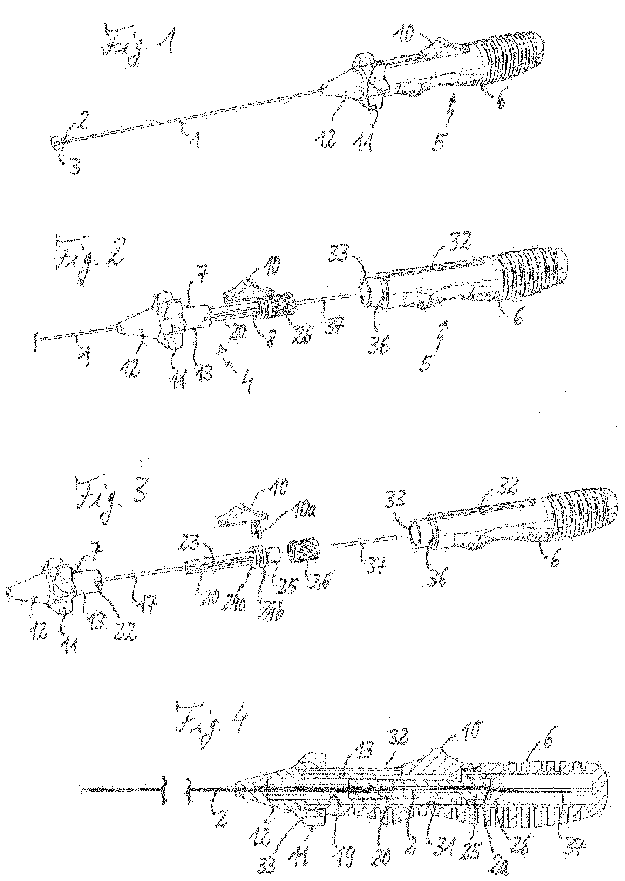

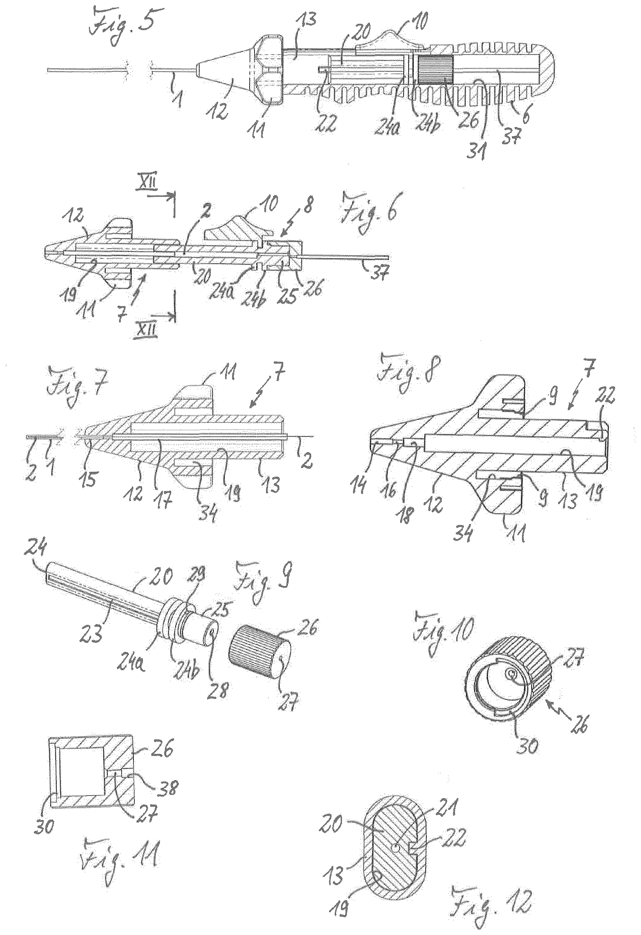

Das in den Fig. 1 bis 14 gezeigte, handbetätigte Funktionsschlauchinstrument ist zur Verwendung als Steinfangkorbinstrument eingerichtet und beinhaltet, wie üblich, zwei langgestreckte Funktionsteile in Form eines schlauchförmigen Funktionsteils 1, kurz Schlauch bezeichnet, und eines sich in diesem axial relativbeweglich erstreckenden drahtförmigen Funktionsteils 2, kurz Funktionsdraht bezeichnet. Wegen seiner Zugkraftfunktionalität wird der Funktionsdraht 2 auch als Zugdraht bezeichnet. An einem vorderen, distalen Ende weist der Funktionsdraht 2 ein faltbares Drahtkörbchen 3 auf, um Steine oder dgl. zum Beispiel in Gewebehohlräumen eines Patienten einfangen und extrahieren zu können. Dies stellt bei diesem Instrument die Nutzfunktion dar. Der Schlauch 1 und der Funktionsdraht 2 können aus beliebigen geeigneten Kunststoff- oder Metallmaterialien bestehen, wie sie für diesen Einsatzzweck bekannt sind, zum Beispiel der Schlauch 1 aus einem Kunststoffmaterial und der Funktionsdraht 2 aus einem superelastischen Metallmaterial.

In einem eingezogenen Funktionszustand befindet sich das Drahtkörbchen 3 eingezogen am distalen Ende des Schlauchs 1 in dessen Innerem. Durch axiales Vorbewegen des Funktionsdrahts 2 relativ zum Schlauch 1 und/oder axiales Zurückbewegen des Schlauchs 1 relativ zum Funktionsdraht 2 gelangt das Drahtkörbchen 3 distal aus dem Schlauch 1 heraus und entfaltet sich aufgrund seiner Eigenelastizität selbsttätig. Fig. 1 zeigt das Instrument in diesem Funktionszustand mit entfaltetem Drahtkörbchen 3. Durch Zugkraftbeaufschlagung des Funktionsdrahts 2 und damit des Drahtkörbchens 3 kann ein eingefangener Stein sicher im Drahtkörbchen 3 eingespannt gehalten werden. Befindet sich kein Partikel im Drahtkörbchen 3, kann es bei Bedarf unter Zusammenfalten wieder vollständig in den Schlauch 1 eingezogen werden, indem der Funktionsdraht 2 relativ zum Schlauch 1 axial zurückbewegt und/oder der Schlauch 1 relativ zum Funktionsdraht 2 axial vorbewegt wird.

Um die Betätigung durch einen Nutzer zwecks Bewirkung der geschilderten axialen Relativbewegung zwischen Schlauch 1 und Funktionsdraht 2 zu ermöglichen, weist das Instrument an seinem hinteren, proximalen Abschnitt eine Betätigungseinheit 4 und einen Bediengriff 5 auf, wobei die Betätigungseinheit 4 lösbar mit dem Bediengriff 5 koppelbar ist, speziell mit einem Griffkörper 6 des Bediengriffs 5. Die Betätigungseinheit 4 umfasst ein erstes Betätigungsteil 7 und ein zweites Betätigungsteil 8, wobei die beiden Betätigungsteile 7, 8 axial relativbeweglich miteinander gekoppelt sind. Eines der beiden Betätigungsteile 7, 8 weist eine Schlauchfixierung auf, durch die das schlauchförmige Funktionsteil 1 an ihm fixiert ist. Das andere der beiden Betätigungsteile 7, 8 weist eine Drahtfixierung auf, durch die das drahtförmige Funktionsteil 2 an ihm fixiert ist. Das erste Betätigungsteil 7 beinhaltet ein Axialfixierelement 9, mit dessen Hilfe es am Bediengriffkörper 6 axial fixierbar ist. Das zweite Betätigungsteil 8 beinhaltet ein fingerbetätigtes Bedienschieberelement 10.

In der speziellen Ausführung gemäß den Fig. 1 bis 14 umfasst das erste Betätigungsteil 7 ein fingerbetätigtes Bediendrehelement 11 und einen distalen Konusabschnitt 12 sowie einen proximalen Führungshülsenabschnitt 13, d.h. einen als Führungshülse 13 fungierenden Abschnitt, wobei es im gezeigten Beispiel einteilig ausgeführt ist, z.B. als Kunststoffspritzgussteil.

Beim Instrument der Fig. 1 bis 14 ist die Schlauchfixierung am ersten Betätigungsteil 7 vorgesehen, wozu es am distalen Ende seines distalen Konusabschnitts 12 eine Schlauchaufnahmebohrung 14 aufweist, in die der Schlauch 1 mit seinem proximalen Ende eingefügt und zum Beispiel durch eine Verklebung 15 als Schlauchfixierung gehalten ist. In proximaler Richtung schließt sich an die Schlauchaufnahmebohrung 14 eine Drahtdurchführungsbohrung 16 an, durch die hindurch sich der Funktionsdraht 2 weiter in proximaler Richtung durch das erste Betätigungsteil 7 hindurch erstreckt, wenn die beiden Funktionsteile 1 und 2 an der Betätigungseinheit 4 montiert sind. Im hülsenförmigen Führungsabschnitt 13 des ersten Betätigungsteils 7 ist optional ein Versteifungsröhrchen 17 vorgesehen, das mit seinem distalen Ende in eine zugehörige Aufnahmebohrung 18 fixiert eingefügt ist, die im ersten Betätigungsteil 7 proximal anschließend an die Drahtdurchführungsbohrung 16 vorgesehen ist. Das Versteifungsröhrchen 17 erstreckt sich vorzugsweise über die gesamte Länge des Führungshülsenabschnitts 13 proximal nach hinten. Optional kann das Versteifungsröhrchen 17 einteilig mit dem übrigen ersten Betätigungsteil 7 gefertigt sein, z.B. in Kunststoffspritzgusstechnik.

Das zweite Betätigungsteil 8 beinhaltet ein Führungselement, das dafür eingerichtet ist, in einer Führungshülse des ersten Betätigungsteils 7 drehfest und axialbeweglich geführt zu werden. Dazu ist beim Instrument der Figuren 1 bis 14 die Führungshülse durch den Führungshülsenabschnitt 13 und einen daran distal anschließenden axialen Bereich des ersten Betätigungsteils 7 gebildet, um eine zugehörige Führungs- bzw. Aufnahmebohrung 19 zu bilden. Die Bohrung 19 erstreckt sich längs des Führungshülsenabschnitts 13 und vorzugsweise axial durch das Bediendrehelement 11 hindurch und bevorzugter noch bis zu einer vorgebbaren Länge in den distalen Konusabschnitt 12 hinein, z.B. bis zu einer Länge von mindestens einem Viertel oder mindestens einem Drittel oder mindestens der Hälfte der axialen Länge des distalen Konusabschnitts 12. Das Hineinerstrecken der Führungsbohrung 19 in den Konusabschnitt 12 ermöglicht bei Bedarf eine besonders kurze Bauform der Betätigungseinheit 7, 8 und des Bediengriffs 5.

Im gezeigten Beispiel des Instruments der Fig. 1 bis 14 ist das Führungselement des zweiten Betätigungsteils 8 durch einen stangenförmigen Führungskörper 20 gebildet, der in der Ausführung gemäß den Fig. 1 bis 14 einen ovalförmigen Querschnitt aufweist. Passend dazu weist die zugehörige Führungsbohrung 19 einen entsprechend ovalförmigen Querschnitt auf, so dass der Stangenkörper 20 im Wesentlichen spielfrei axial beweglich in der Aufnahmebohrung 19 geführt ist. Zur Montage wird der stangenförmige Führungskörper 20 mit seinem distalen Ende voraus proximal von hinten in die zugehörige Aufnahmebohrung 19 eingeschoben. Der Funktionsdraht 2 und das Versteifungsröhrchen 17 werden in einer zentrischen Durchführungsbohrung 21 des stangenförmigen Führungskörpers 20 aufgenommen.

In distaler Richtung begrenzt das Ende der Aufnahmebohrung 19 im distalen Konusabschnitt 12 des ersten Betätigungsteils 7 das Vorbewegen des Führungsstangenkörpers 20 und damit des zweiten Betätigungsteils 8 insgesamt relativ zum ersten Betätigungsteil 7. In proximaler Richtung verhindert eine am proximalen Ende des hülsenförmigen Führungsabschnitts 13 des ersten Betätigungsteils 7 radial federnd ausgebildete Stoppnase bzw. Rastzunge 22 ein unabsichtliches vollständiges Herausbewegen des Führungsstangenkörpers 20 aus dem ersten Betätigungsteil 7. Dazu weist der Führungsstangenkörper 20 eine äußere Axialnut 23 auf, die mit etwas Abstand vor dem distalen Stirnende 24 des Führungsstangenkörpers 20 endet und in welche die beim Einschieben des Führungsstangenkörpers 20 in die Aufnahme 19 federelastisch radial nach außen gedrückte Nase 22 eingreift. Auf diese Weise umfassen die beiden Betätigungsteile 7, 8 Endanschläge zur beidseitigen Begrenzung der axialen Relativbewegung von Führungshülse und Führungselement.

Damit bleiben die beiden Betätigungsteile 7, 8 vormontiert aneinander gehalten, auch wenn sie noch nicht am Bediengriff 5 montiert sind oder von diesem wieder gelöst worden sind. Der ovale Querschnitt von Führungsstangenkörper 20 des zweiten Betätigungsteils 8 und zugehöriger Führungsaufnahme 19 im ersten Betätigungsteil 7 stellt ohne weitere Maßnahmen eine drehfeste Kopplung des Führungsstangenkörpers 20 des zweiten Betätigungsteils 8 mit dem ersten Betätigungsteil 7 bereit.

Das Bedienschieberelement 10 ist axial fixiert und drehbeweglich am Führungsstangenkörper 20 gehalten. Dazu ist letzterer in einem proximalen Bereich mit zwei axial beabstandeten Ringflanschen 24a, 24b versehen, in deren Zwischenraum das Bedienschieberelement 10 mit einer halbkreisförmigen, an seiner Unterseite angeformten Lagergabel 10a eingreift. Dadurch ist das Bedienschieberelement 10 axial am Führungsstangenkörper 20 fixiert, während der Führungsstangenkörper 20 gedreht werden kann, ohne dass sich dadurch das Bedienschieberelement 10 zwangsweise mitdreht. Vorteilhaft kann vorgesehen sein, dass sich die Lagergabel 10a über etwas mehr als 180° in Umfangsrichtung erstreckt und als Schnapp- bzw. Clipselement fungiert, das auf den in diesem Bereich kreisförmigen Querschnitt des Führungsstangenkörpers 20 aufgeschnappt bzw. aufgeclipst und auf diese Weise verliersicher am Führungsstangenkörper 20 gehalten wird.

Am proximalen Ende des Führungsstangenkörpers 20 hinter den Ringflanschen 24a, 24b ist ein Drahtfixierkörper 25 ausgebildet, der als ein erstes Drahtfixierteil fungiert, das mit einem zweiten Drahtfixierteil 26 zur Bildung einer Drahtfixierung zusammenwirkt, durch die das drahtförmige Funktionsteil 2 mit seinem proximalen Ende am zweiten Betätigungsteil 8 lösbar fixiert werden kann. Im gezeigten Ausführungsbeispiel der Fig. 1 bis 14 ist das erste Drahtfixierteil 25 durch einen rohrförmigen Endabschnitt des zweiten Betätigungsteils 8 realisiert, und das zweite Drahtfixierteil 26 ist durch einen daran ansteckbaren Fixierstutzen gebildet. Bei diesem Fixierstutzen kann es sich beispielsweise, wie gezeigt, um eine einseitig offene Fixierhülse handeln, die an ihrer geschlossenen Stirnseite mit einer zur Hülsenlängsachse exzentrischen Durchführungsöffnung 27 versehen ist. Korrespondierend dazu mündet die Drahtdurchführungsbohrung 21 des Führungsstangenkörpers 20 an dessen proximaler Stirnseite mit einer exzentrischen Drahtdurchführungsöffnung 28 aus, wobei beide korrespondierenden Drahtfixieröffnungen 27, 28 um das gleiche Maß gegenüber einer als Fixierdrehachse fungierenden Längsmittenachse versetzt sind. Dies hat zur Folge, dass die beiden Öffnungen 27, 28 in einer bestimmten Drehwinkelstellung des hülsenförmigen Fixierstutzens 26 relativ zum korrespondierenden Fixierrohrstutzen 25 miteinander fluchten, was eine Drahtfreigabestellung der so gebildeten Drahtfixierung darstellt. Im gezeigten Beispiel sind beide Drahtführungsöffnungen 27, 28 als umfangsseitig geschlossene Öffnungen ausgeführt.

In dieser Drahtfreigabestellung kann das proximale Ende des drahtförmigen Funktionsteils 2 durch die beiden Öffnungen 27, 28 hindurchgeschoben werden, zur Montage proximal nach hinten, zur Demontage distal nach vorn. Der hülsenförmige Fixierstutzen 26 wird mit seiner offenen Stirnseite auf den Fixierrohrstutzen 25 aufgesteckt und kann in dieser Lage relativ zu diesem gedreht werden. Speziell kann er von der Drahtfreigabestellung in eine Drahtklemmstellung verdreht werden, in der die beiden Durchführungsöffnungen 27, 28 außer Flucht sind. Das durch die beiden Öffnungen 27, 28 durchgeschobene proximale Endstück des drahtförmigen Funktionsteils 2 wird dadurch entsprechend verformt und durch seinen daraus resultierenden verformten, umgebogenen Verlauf im Bereich der beiden außer Flucht befindlichen Öffnungen 27, 28 klemmend am zweiten Betätigungsteil 8 fixiert.

Es zeigt sich, dass allein die Verformung bzw. Verbiegung, die der durchgeführte Endabschnitt des drahtförmigen Funktionsteils 2 durch das Verdrehen der beiden exzentrischen Durchführungsöffnungen 27, 28 erfährt, eine ausreichend hohe Klemmwirkung bereitstellt, die das drahtförmige Funktionsteil 2 sicher am zweiten Betätigungsteil 8 fixiert hält, wobei diese Drahtfixierung den geforderten Belastungen problemlos standhält. Dies gilt insbesondere auch bei Verwendung superelastischer Drahtmaterialien für das drahtförmige Funktionsteil 2.

Um die Drahtfreigabestellung und die Drahtklemmstellung festzulegen und ein übermäßiges Verdrehen des hülsenförmigen Fixierstutzens 26 gegenüber dem Fixierrohrstutzen 25 zu vermeiden, sind an diesen beiden Teilen geeignete Drehbegrenzungsanschläge ausgebildet, im gezeigten Fall am Fixierrohrstutzen 25 eine Stoppnase 29 und am hülsenförmigen Fixierstutzen 26 eine sich über einen entsprechenden Winkelbereich erstreckende Führungsnut 30, innerhalb der sich die Stoppnase 29 bewegen kann, z.B. über einen Winkelbereich zwischen 90° und 270°.

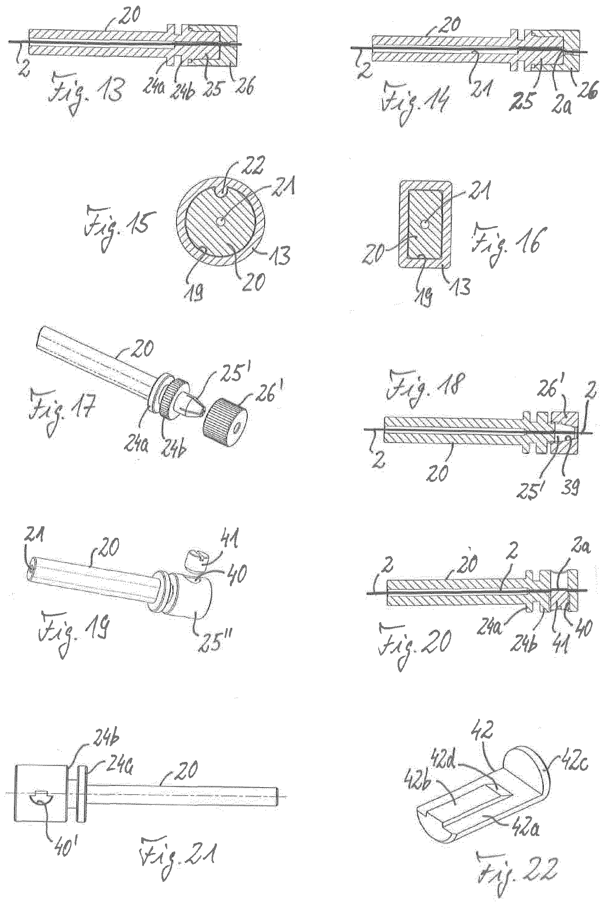

Die Fig. 13 und 14 veranschaulichen die Drahtfixierung durch die beiden Drahtfixierteile 25, 26, wobei Fig. 13 die Drahtfreigabestellung zeigt, in der die Drahtdurchführungsöffnungen 27, 28 fluchten, während Fig. 14 die Drahtklemmstellung zeigt, in welcher die Drahtdurchführungsöffnungen 27, 28 außer Flucht sind und das proximale Ende des drahtförmigen Funktionsteils 2 dadurch einen festklemmenden, fixierenden Biegungs- bzw. Knickverlauf 2a erhält. Letzter ist durch Zurückdrehen des hülsenförmigen Fixierstutzens 26 von der Drahtklemmstellung in die Drahtfreigabestellung reversibel, wonach das drahtförmige Funktionsteil 2 aus dem zweiten Betätigungsteil 8 wieder herausgezogen werden kann.

Die vormontierte Instrumenteneinheit aus der Betätigungseinheit mit dem ersten Betätigungsteil 7, dem zweiten Betätigungsteil 8, dem am ersten Betätigungsteil 7 fixierten schlauchförmigen Funktionsteil 1 und dem am zweiten Betätigungsteil 8 fixierten drahtförmigen Funktionsteil 2 kann in sehr einfacher Weise lösbar am Bediengriffkörper 6 montiert werden. Der Bediengriffkörper 6 bildet eine Griffschale, die speziell zum ergonomischen Umgreifen mit einer Hand geformt ist und in ihrem Inneren eine distal nach vorne offene Aufnahme 31 zum Aufnehmen der Betätigungseinheit 7, 8 aufweist. An einer Oberseite ist am Griffkörper 6 eine Schlitzführung 32 ausgebildet, in welcher das Bedienschieberelement 10 axialbeweglich geführt ist, wobei es durch diese Schlitzführung 32 gleichzeitig drehfest am Griffkörper 6 gehalten ist.

Das erste Betätigungsteil 7 ist drehbeweglich und axial fixiert am Bediengriffkörper 6 gehalten. Dazu weist der Bediengriffkörper 6 an seinem Vorderende einen axialen Ringfortsatz 33 auf, während korrespondierend am ersten Betätigungsteil 7 radial zwischen dem außenliegenden Drehelement 11 und dem inneren Führungsabschnitt für den Führungsstangenkörper 20 eine Ringausnehmung 34 ausgebildet ist, in die der axiale Ringfortsatz 33 des Bediengriffkörpers 6 eingesteckt werden kann. Am hinteren Ende des axialen Fortsatzes 33 weist der Griffkörper 6 eine als Rastnut fungierende umlaufende Ringnut 36 auf, in welche das Axialfixierelement 9 lösbar verrastend eingreift, das im gezeigten Beispiel der Figuren 1 bis 14 von einer oder mehreren, in Umfangsrichtung verteilt angeordneten Rastzungen 9 gebildet ist, die radial nach innen in die Ringaufnahmenut 34 hinein ragen. Damit ist das erste Betätigungsteil 7 und mit diesem die gesamte vormontierte Baueinheit aus erstem und zweitem Betätigungsteil 7, 8 sowie schlauchförmigem und drahtförmigem Funktionsteil 1, 2 lösbar in axialer Richtung fixiert am Bediengriffkörper 6 montierbar. Die Ringnut 36 fungiert somit als Koppelelement zum axial fixierten Ankoppeln des ersten Betätigungsteils 7 an den Griffkörper 6. Alternativ sind die Rastzungen 9 einerseits und die Ringnut 36 andererseits in ihrer Position vertauscht, d.h. die Ringnut 36 ist dann am ersten Betätigungsteil 7 ausgebildet, und die Rastzungen 9 sind am Bediengriffkörper 6 vorgesehen. Dies vermeidet, dass die Rastzungen 9 eventuell störend in die Schlitzführung 32 des Griffkörpers 6 gelangen.

Im montierten Zustand kann das erste Betätigungsteil 7 relativ zum Griffkörper 6 verdreht werden, wobei von dieser Drehbewegung auch der Führungsstangenkörper 20 und die Drahtfixierung 25, 26 mitgenommen werden, während das Bedienschieberelement 10 im Führungsschlitz 32 gehalten wird, ohne sich mitzudrehen. Der Benutzer kann diese Drehung durch entsprechende Betätigung des fingerbetätigten Bediendrehelements 11 zum Beispiel mit seinem Daumen bewirken, während er den Griffkörper 6 mit einer Hand umgreift. Er kann dadurch das schlauchförmige und das drahtförmige Funktionsteil 1, 2 synchron relativ zum Bediengriff 5 drehen.

Des Weiteren kann der Benutzer insbesondere ebenfalls mit seinem Daumen das fingerbetätigte Bedienschieberelement 10 betätigen, indem er es relativ zum Griffkörper 6 axial vor bzw. zurück bewegt. Dadurch bewirkt er im gezeigten Beispiel der Fig. 1 bis 14 ein axiales Vorbewegen des drahtförmigen Funktionsteils 2 relativ zum Griffkörper 6 und dem an ihm über das erste Betätigungsteil 7 axial fixierten schlauchförmigen Funktionsteil 1. So kann er das Drahtkörbchen 3 am distalen Instrumentenende aus dem Schlauch 2 nach vorn heraus bewegen und entfalten bzw. wieder in den Schlauch 2 zusammenfaltend hinein bewegen. Bei dieser Axialbewegung bewegt sich das zweite Betätigungsteil 8 vor und zurück, wobei es im ersten Betätigungsteil 7 durch den Führungsstangenkörper 20 und in der Griffkörperausnehmung 31 durch die beiden Radialflansche 24a, 24b geführt ist, wozu letztere einen dem Durchmesser der Griffkörperausnehmung 31 entsprechenden Außendurchmesser aufweisen und solchermaßen als Führungs- bzw. Zentrierring fungieren. Zur axialen Hubbegrenzung ist optional ein Distanzstab 37 vorgesehen, der vom proximalen Ende des zweiten Betätigungsteils 8 proximal nach hinten absteht und die Axialbewegung nach hinten durch Anschlagen gegen das hintere Ende der Griffkörperausnehmung 31 begrenzt. Der Distanzstab 37 kann zum Beispiel in einer Aufnahmebohrung 38 des hülsenförmigen Drahtfixierstutzens 26 montiert sein, die als verbreiterte Verlängerung der exzentrischen Drahtdurchführungsöffnung 27 gebildet ist.

Wie die obigen Erläuterungen deutlich machen, eignet sich das Instrument der Fig. 1 bis 14 für eine komfortable Handbetätigung, insbesondere eine Einhandbedienung, bei welcher der Benutzer nur eine Hand zur Instrumentenbetätigung benötigt, wobei er den ergonomisch geformten Griffkörper 6 mit der Hand bequem umgreifen kann und sowohl das Bedienschieberelement 10 als auch das Bediendrehelement 11 zum Beispiel allein mit seinem Daumen betätigen kann.

Im Folgenden wird auf einige beispielhaft gezeigte Ausführungsvarianten des Instruments der Fig. 1 bis 14 eingegangen, wobei zum besseren Verständnis für identische und funktionell entsprechende Elemente gleiche Bezugszeichen verwendet sind und insoweit auf die obigen Ausführungen zur Ausführungsform der Fig. 1 bis 14 verwiesen werden kann, falls nichts anderes gesagt.

Die Fig. 15 und 16 veranschaulichen zwei Ausführungsvarianten bzgl. der Querschnittsform des Führungsstangenkörpers 20 und der korrespondierenden Führungsaufnahme 19 im ersten Betätigungsteil 7. Im Ausführungsbeispiel von Fig. 15 ist statt eines ovalen ein kreisrunder Querschnitt gewählt. In diesem Fall dient die Führungsnase bzw. Rastzunge 22 gleichzeitig zur drehfesten Verbindung des Führungsstangenkörpers 20 des zweiten Betätigungsteils 8 mit dem ersten Betätigungsteil 7. Bei der Ausführungsvariante von Fig. 16 ist statt des ovalen ein rechteckförmiger Querschnitt von Führungsstangenkörper 20 und zugehöriger Führungsaufnahme 19 gewählt.

Die Fig. 17 bis 22 veranschaulichen verschiedene Ausführungsvarianten für die Drahtfixierung des drahtförmigen Funktionsteils 2 am zweiten Betätigungsteil 8 und speziell am proximalen Endabschnitt des Führungsstangenkörpers 20. Im Ausführungsbeispiel der Fig. 17 und 18 ist dazu am proximalen Ende des Führungsstangenkörpers 20 ein erstes Drahtfixierteil in Form eines axial geschlitzten und sich proximal nach hinten konisch verengenden Rohrstutzens 25' ausgebildet, mit dem ein hülsenförmiger Fixierstutzen 26' zusammenwirkt, der eine sich korrespondierend verengende Rohrstutzenaufnahme 39 aufweist. Durch Aufstecken der Fixierhülse 26' auf den Fixierrohrkonus 25' wird letzterer in der Aufnahme 39 radial zusammengedrückt, wodurch er den durchgeführten proximalen Endabschnitt des drahtförmigen Funktionsteils 2 klemmend festhält.

Im Ausführungsbeispiel der Fig. 19 und 20 beinhaltet die Drahtfixierung einen mit einer Radialbohrung 40 versehenen, rohrförmigen Endabschnitt 25" des Führungsstangenkörpers 20. Als weiteres Drahtfixierteil beinhaltet diese Drahtfixierung einen Fixierstift 41, der in die Radialbohrung 40 eingeschraubt werden kann, bis er eine in Fig. 20 gezeigte Drahtklemmstellung erreicht, in welcher er auf den mittig durchgeführten Funktionsdraht stößt und diesen im Bereich der Radialbohrung 40 mitnimmt und umbiegt, so dass das drahtförmige Funktionsteil 2 wiederum am als Fixierkörper fungierenden Führungsstangenkörper 20 mit einem umgeformten bzw. umgebogenen/umgeknickten proximalen Endabschnitt 2a festgeklemmt gehalten ist.

Im Ausführungsbeispiel der Fig. 21 und 22 ist die Drahtfixierung ähnlich realisiert wie im Beispiel der Fig. 19 und 20, wobei statt des schraubbaren Fixierstifts 41 ein Steckfixierstift 42 vorgesehen ist, der einen von einem Kopfteil 42c abstehenden Einsteckkörper 42a mit etwa halbkreisförmigem Querschnitt beinhaltet, auf dem über eine gewisse Länge eine Auflagefläche 42b mit keilförmiger Anlauffläche 42d ausgebildet ist. Dieser Stift 42 kann in eine Radialbohrung 40' mit entsprechend modifizierter Querschnittsform eingesteckt werden, die wiederum am rohrförmigen Endabschnitt 25" des Führungsstangenkörpers 20 ausgebildet ist. Durch Anlaufen gegen die Keilfläche 42d kann der durch den Führungsstabkörper 20 hindurchgeführte Funktionsdraht 2 in seinem proximalen Endabschnitt entsprechend wie im Ausführungsbeispiel der Fig. 19 und 20 umgebogen/umgeformt werden, wobei er dann mit seinem umgebogenen bzw. umgeformten Bereich auf der Auflagefläche 42b aufliegt.

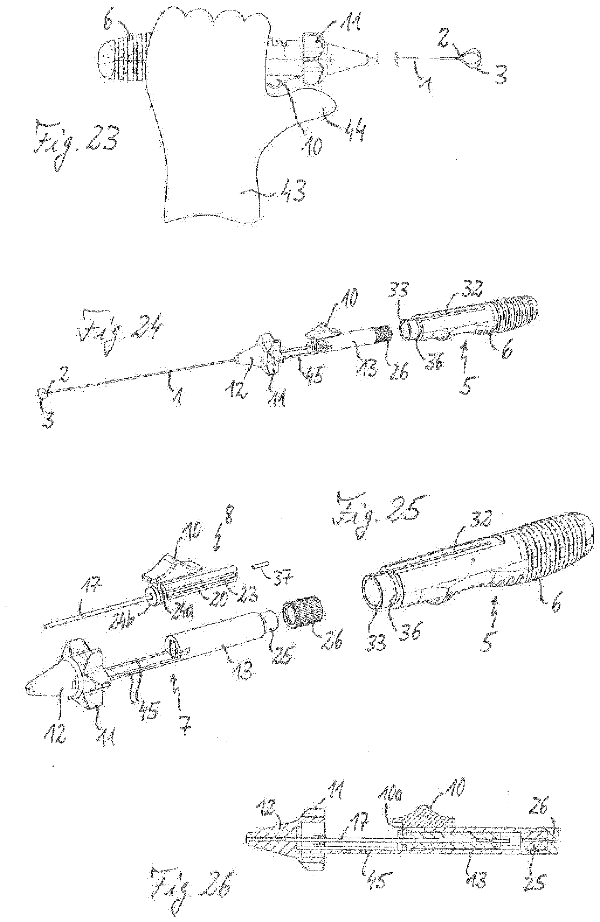

Fig. 23 illustriert die angesprochene komfortable Bedienbarkeit des Instruments in den Ausführungsvarianten der Figuren 1 bis 22 unter Zuhilfenahme nur einer Hand des Benutzers, mit der er den ergonomisch geformten Griffkörper 6 umgreift, wobei er vorzugsweise mit seinem Daumen 44 sowohl das Bedienschieberelement 10 als auch das Bediendrehelement 11 betätigen kann.

In den Ausführungsbeispielen der Figuren 1 bis 22 ist das schlauchförmige Funktionsteil 1 am ersten Betätigungsteil 7 fixiert, während das drahtförmige Funktionsteil 2 am Betätigungsteil 8 fixiert ist. Die Fig. 24 bis 26 veranschaulichen eine Ausführungsvariante, bei denen das schlauchförmige Funktionsteil 1 am zweiten Betätigungsteil 8 fixiert ist, während das drahtförmige Funktionsteil 2 am ersten Betätigungsteil 7 fixiert ist.

Dazu ist das erste Betätigungsteil 7 dahingehend modifiziert, dass der Führungshülsenabschnitt 13 axial vom Bediendrehelement 11 und dem daran distal anschließenden Konusabschnitt 12 beabstandet und mit dem Bediendrehelement 11 über einen Stegabschnitt 45 verbunden ist, der vorzugsweise einstückig mit dem Bediendrehelement 11 und dem Führungshülsenabschnitt 13 gebildet ist. Die Drahtfixierung mit dem Fixierrohrstutzen 25 und dem darauf aufsteckbaren, hülsenförmigen Fixierstutzen 26 ist in diesem Fall am proximalen Ende des Fixierhülsenabschnitts 13 des ersten Betätigungsteils 7 ausgebildet, so dass dort das drahtförmige Funktionsteil 2 am ersten Betätigungsteil 7 fixiert ist.

Das zweite Betätigungsteil 8 ist dahingehend modifiziert, dass die beabstandeten Ringflansche 24a, 24b zur drehbeweglichen Axialfixierung des Bedienschieberelements 10 an einem vorderen, distalen Endabschnitt des Führungsstangenkörpers 20 ausgebildet sind und der axiale Führungsschlitz 23 mit geringfügigem Abstand vor dem proximalen Stirnende des Führungsstangenkörpers 20 endet. Das schlauchförmige Funktionsteil 1 ist durch eine entsprechende zentrische Bohrung im Konusabschnitt 12 und im Bediendrehelement 11 durch selbige hindurch geführt und mit seinem proximalen Ende am und im Führungsstangenkörper 20 fixiert, vorzugsweise in etwa auf Höhe der Ringflansche 24a, 24b, zum Beispiel durch eine Verklebung.

Somit kann bei dieser Ausführungsvariante der Fig. 24 bis 26 der Benutzer durch axiales Betätigen des Bedienschieberelements 10 den Schlauch 1 aktiv gegenüber dem Funktionsdraht 2 vor und zurück bewegen, während der Funktionsdraht 2 gegenüber dem Bediengriff 5 axial unbewegt bleibt. Auf diese Weise bleibt der Funktionsdraht 2 axial unbewegt an Ort und Stelle, wenn der Benutzer den Schlauch 1 axial zurückbewegt und dadurch das Drahtkörbchen 3 aus dem Schlauch 2 herausgelangt und sich entfaltet. Entsprechend bleibt das Drahtkörbchen 3 axial an Ort und Stelle, wenn der Benutzer den Schlauch 2 wieder vorbewegt, um das Drahtkörbchen 3 zusammenzuziehen. Dies kann für medizinische Steinfanganwendungen vorteilhaft sein.

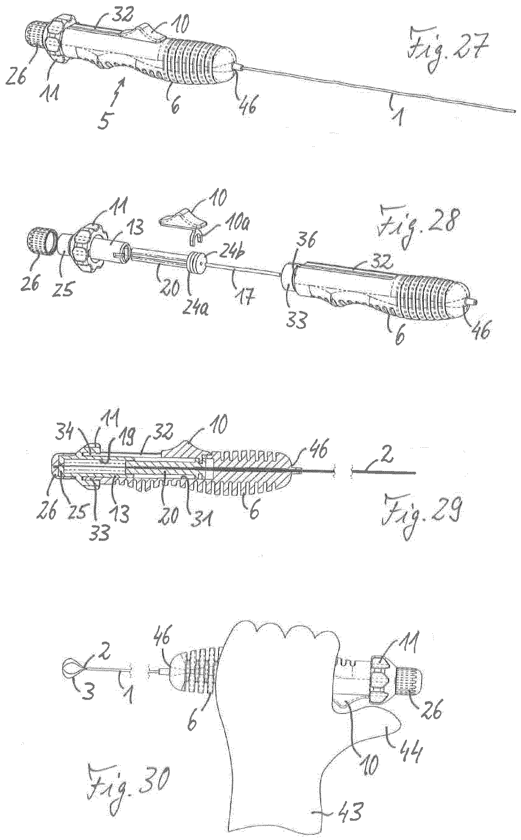

Die Fig. 27 bis 30 veranschaulichen eine erfindungsgemäße Realisierung, bei der das handbetätigte Funktionsschlauchinstrument in einer zu derjenigen der oben beschriebenen Beispiele der Fig. 1 bis 26 umgekehrten Griffrichtung vom Benutzer gegriffen wird, wie dies durch Vergleich der Fig. 30 mit der Fig. 23 deutlich wird. Bei der Ausführungsvariante der Fig. 27 bis 30 ist das Bediendrehelement 11 am proximalen Ende statt am distalen Ende des insofern modifizierten Bediengriffkörpers 6 angeordnet. Am distalen Ende weist der Bediengriffkörper 6 einen Austrittskonus 46 auf, aus dem das schlauchförmige und das drahtförmige Funktionsteil 1, 2 in distaler Richtung austreten. Das zweite Betätigungsteil 8 ist wie beim Ausführungsbeispiel der Fig. 23 bis 26 gestaltet, wobei wiederum das schlauchförmige Funktionsteil 1 am Fixierstangenkörper 20 fixiert ist.

In der Ausführungsvariante der Fig. 27 bis 30 ragt der Führungshülsenabschnitt 13 proximal und distal über den Bereich des Bediendrehelements 11 hinaus, wobei sein proximales Ende wie beim Ausführungsbeispiel der Fig. 23 bis 26 als das rohrstutzenförmige Drahtfixierteil 25 gestaltet ist, mit dem das andere Drahtfixierteil in Form des hülsenförmigen Fixierstutzens 26 zusammenwirkt, um die Fixierung bereitzustellen, mit welcher das drahtförmige Funktionsteil 2 am ersten Betätigungsteil 7 fixiert ist.

Wie aus Fig. 30 zu erkennen, erstrecken sich bei dieser Ausführungsvariante das schlauchförmige und das drahtförmige Funktionsteil 1, 2, wenn der Benutzer mit seiner Hand 43 den Bediengriffkörper 6 greift und mit seinem Daumen 44 das Bediendrehelement und das Bedienschieberelement 10 betätigen kann, von seiner daumenabgewandten Handseite aus dem Bediengriff 5 heraus, während umgekehrt im Fall von Fig. 23 die beiden Funktionsteile 1, 2 auf der daumenzugewandten Handseite aus dem Bediengriff 5 austreten.

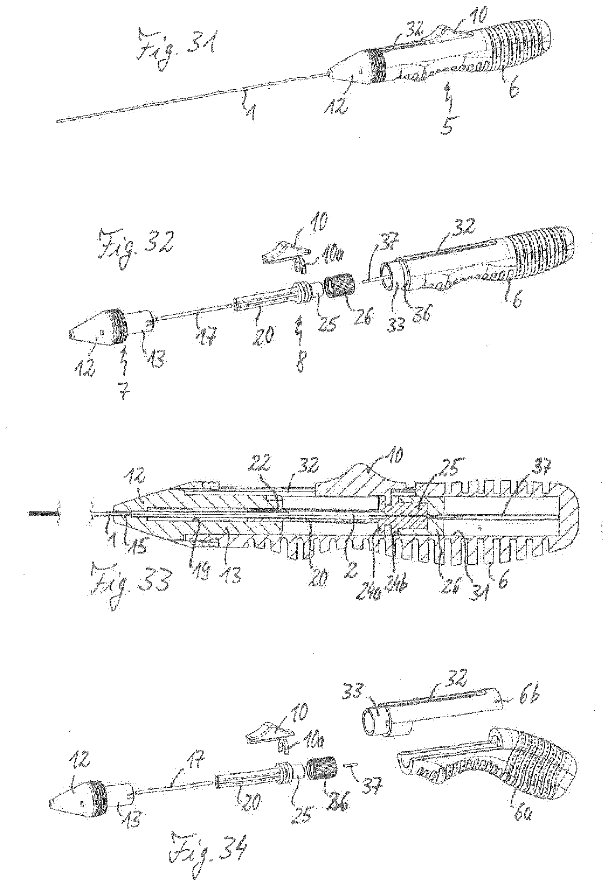

Die Fig. 31 bis 33 veranschaulichen ein Ausführungsbeispiel, das demjenigen der Fig. 1 bis 14 mit der Ausnahme entspricht, dass das erste Betätigungsteil 7 ohne fingerbetätigtes Bediendrehelement 11 realisiert ist. Diese Ausführungsvariante eignet sich somit für Anwendungen, bei denen kein Bedarf an einer aktiven Verdrehung des ersten Betätigungsteils 7 gegenüber dem Griffkörper 6 besteht. Im Übrigen weist das Instrument der Fig. 31 bis 33 die gleichen vorteilhaften Merkmale und Eigenschaften auf, wie sie oben zum Ausführungsbeispiel der Figuren 1 bis 14 erläutert sind, worauf verwiesen werden kann.

Fig. 34 zeigt eine Ausführungsvariante, die derjenigen der Fig. 31 bis 33 mit der einzigen Modifikation entspricht, dass der Bediengriff 6 zweiteilig gestaltet ist mit einem Griffgrundkörper bzw. Griffbasisteil 6a und einem mit diesem lösbar zu verbindenden Aufsatzkörper bzw. Griffkoppelteil 6b, wobei an letzterem die Betätigungseinheit 7,8 montiert wird. Dies kann die Montage des Instruments weiter vereinfachen. Eine solche zweiteilige Griffausführung kann auch bei den anderen erwähnten Ausführungsformen der Erfindung Verwendung finden.

Wie die obige Beschreibung vorteilhafter Ausführungsbeispiele deutlich macht, stellt die Erfindung ein Funktionsschlauchinstrument bereit, das mit relativ wenig Aufwand herstellbar und komfortabel mit einer Hand bedienbar ist, wobei seine Komponenten in einfacher Weise aneinander lösbar montiert und wieder voneinander demontiert werden können. Die Erfindung eignet sich nicht nur wie beschrieben für medizinische Steinfangkorbinstrumente, sondern auch für andere endoskopische Funktionsschlauchinstrumente und für beliebige andere Anwendungen, die ein Instrument der betrachteten Art benutzen, das über einen Bediengriff handbetätigt werden soll.

## Patentansprüche

1. Handbetätigtes Funktionsschlauchinstrument, insbesondere endoskopisches Funktionsschlauchinstrument, mit
- einem schlauchförmigen Funktionsteil (1) und einem sich in diesem erstreckenden drahtförmigen Funktionsteil (2), wobei die beiden Funktionsteile axial relativbeweglich sind, um an einem distalen Ende eine Nutzfunktion auszuüben, und
- einer zum lösbaren Koppeln mit einem Bediengriffkörper eingerichteten Betätigungseinheit, die an einem proximalen Endabschnitt der Funktionsteile angeordnet ist und ein erstes und zweites Betätigungsteil (7, 8) umfasst, die axial relativbeweglich miteinander gekoppelt sind, wobei
- eines der beiden Betätigungsteile eine Schlauchfixierung aufweist, durch die das schlauchförmige Funktionsteil an ihm fixiert ist, und das andere der beiden Betätigungsteile eine Drahtfixierung aufweist, durch die das drahtförmige Funktionsteil an ihm fixiert ist,
- das erste Betätigungsteil (7) ein Axialfixierelement (9) zum axialen Fixieren des ersten Betätigungsteils am Bediengriffkörper aufweist und
- das zweite Betätigungsteil (8) ein fingerbetätigtes Bedienschieberelement (10) aufweist,
**dadurch gekennzeichnet, dass**
- die Drahtfixierung zwei gegeneinander um eine Längsachse von einer Drahtfreigabestellung in eine Drahtklemmstellung verdrehbare Drahtfixierteile (25, 26) mit je einer exzentrischen axialen Drahtdurchführungsöffnung (27, 28) umfasst, wobei die beiden Durchführungsöffnungen axial hintereinander angeordnet sind und in der Drahtfreigabestellung fluchten und in der Drahtklemmstellung außer Flucht sind.

2. Funktionsschlauchinstrument nach Anspruch 1, weiter **dadurch gekennzeichnet, dass** das erste Betätigungsteil (7) ein fingerbetätigtes Bediendrehelement (11) aufweist.

3. Funktionsschlauchinstrument nach Anspruch 1 oder 2, weiter **dadurch gekennzeichnet, dass** das erste Betätigungsteil (7) einen distalen Konusabschnitt (12) mit einer axialen Ausnehmung (19) aufweist, in die das zweite Betätigungsteil (8) wenigstens partiell einschiebbar ist.

4. Funktionsschlauchinstrument nach einem der Ansprüche 1 bis 3, weiter **dadurch gekennzeichnet, dass** das zweite Betätigungsteil einen mit dem ersten Betätigungsteil drehfest und axialbeweglich gekoppelten Fixierkörper (20) zur Fixierung des zugeordneten Funktionsteils aufweist, wobei das Bedienschieberelement drehbeweglich und axial fixiert mit dem Fixierkörper gekoppelt ist.

5. Funktionsschlauchinstrument nach einem der Ansprüche 1 bis 4, weiter **dadurch gekennzeichnet, dass** eines der beiden Betätigungsteile eine Führungshülse (13) aufweist und das andere Betätigungsteil ein in dieser drehfest und axialbeweglich geführtes Führungselement (20) aufweist.

6. Funktionsschlauchinstrument nach Anspruch 5, weiter **dadurch gekennzeichnet, dass** die beiden Betätigungsteile Endanschläge zur beidseitigen Begrenzung der axialen Relativbewegung von Führungshülse und Führungselement aufweisen.

7. Funktionsschlauchinstrument nach einem der Ansprüche 1 bis 6, weiter **dadurch gekennzeichnet, dass** die beiden Drahtfixierteile durch einen rohrförmigen Endabschnitt des betreffenden Betätigungsteils und einen an diesen ansteckbaren Fixierstutzen gebildet sind.

8. Funktionsschlauchinstrument nach einem der Ansprüche 1 bis 7, weiter **dadurch gekennzeichnet, dass**
- das erste Betätigungsteil die Schlauchfixierung aufweist und das zweite Betätigungsteil die Drahtfixierung aufweist oder
- das erste Betätigungsteil die Drahtfixierung aufweist und das zweite Betätigungsteil die Schlauchfixierung aufweist.

9. Funktionsschlauchinstrument nach einem der Ansprüche 2 bis 8, weiter **dadurch gekennzeichnet, dass**
- das Bediendrehelement distal vor dem Bedienschieberelement angeordnet ist oder
- das Bediendrehelement proximal hinter dem Bedienschieberelement angeordnet ist.

10. Funktionsschlauchinstrument nach einem der Ansprüche 1 bis 9, weiter **gekennzeichnet durch** einen Bediengriff (5) mit einem Griffkörper (6), der zum lösbaren Aufnehmen der Betätigungseinheit (7, 8) eingerichtet ist und ein Koppelelement (36) zum axial fixierten Ankoppeln des ersten Betätigungsteils aufweist.

11. Funktionsschlauchinstrument nach Anspruch 10, weiter **dadurch gekennzeichnet, dass** das Koppelelement des Griffkörpers zum drehbeweglichen Ankoppeln des ersten Betätigungsteils eingerichtet ist.

12. Funktionsschlauchinstrument nach Anspruch 10 oder 11, weiter **dadurch gekennzeichnet, dass** der Griffkörper eine Axialführung für das Bedienschieberelement aufweist.

13. Funktionsschlauchinstrument nach einem der Ansprüche 10 bis 12, weiter **dadurch gekennzeichnet, dass** das Bediendrehelement an einer distalen oder proximalen Stirnseite des Griffkörpers angeordnet ist.

14. Funktionsschlauchinstrument nach einem der Ansprüche 10 bis 13, weiter **dadurch gekennzeichnet, dass** der Griffkörper mehrteilig ausgeführt ist, wobei er ein Griffbasisteil (6a) und ein mit diesem lösbar verbindbares Griffkoppelteil (6b) umfasst und wobei das Griffkoppelteil zum lösbaren Aufnehmen der Betätigungseinheit (7, 8) eingerichtet ist und das Koppelelement zum Ankoppeln des ersten Betätigungsteils aufweist.

15. Funktionsschlauchinstrument nach einem der Ansprüche 1 bis 14, weiter **dadurch gekennzeichnet, dass** wenigstens eine der beiden Drahtführungsöffnungen (27, 28) eine umfangsseitig geschlossene Öffnung ist.

## Claims

1. Hand-operated functional hose instrument, preferably endoscopic functional hose instrument, comprising
- a hose-like functional part (1) and a wire-like functional part (2) which extends in said hose-like functional part, wherein the two functional parts are axially relatively movable in order to perform a useful function at a distal end, and
- an operating unit designed for releasable coupling to an operator control handle body, which unit is arranged on a proximal end section of the functional parts and comprises a first and a second operating part (7, 8) which are coupled to one another in an axially relatively movable manner, wherein
- one of the two operating parts has a hose fixing arrangement by way of which the hose-like functional part is fixed to it, and the other of the two operating parts has a wire fixing arrangement by way of which the wire-like functional part is fixed to it,
- the first operating part (7) has an axial fixing element (9) for axially fixing the first operating part to the operator control handle body, and
- the second operating part (8) has a finger-operated operator control slide element (10),
**characterized in that**
- the wire fixing arrangement comprises two wire fixing parts (25, 26) which can be rotated in relation to one another about a longitudinal axis from a wire release position to a wire clamping position and each have an eccentric axial wire passage opening (27, 28), wherein the two passage openings are arranged axially one behind the other and are in alignment in the wire release position and are out of alignment in the wire clamping position.

2. Functional hose instrument according to claim 1, further **characterized in that** the first operating part (7) has a finger-operated operator control rotary element (11).

3. Functional hose instrument according to claim 1 or 2, further **characterized in that** the first operating part (7) has a distal cone section (12) with an axial recess (19) into which the second operating part (8) can be at least partially inserted.

4. Functional hose instrument according to any one of claims 1 to 3, further **characterized in that** the second operating part has a fixing body (20), which is coupled to the first operating part in a rotationally fixed and axially movable manner, for the purpose of fixing the associated functional part, wherein the operator control slide element is coupled to the fixing body in a rotationally movable and axially fixed manner.

5. Functional hose instrument according to any one of claims 1 to 4, further **characterized in that** one of the two operating parts has a guide sleeve (13) and the other operating part has a guide element (20) which is guided in said guide sleeve in a rotationally fixed and axially movable manner.

6. Functional hose instrument according to claim 5, further **characterized in that** the two operating parts have end stops for limiting the axial relative movement of the guide sleeve and the guide element on both sides.

7. Functional hose instrument according to any one of claims 1 to 6, further **characterized in that** the two wire fixing parts are formed by a tubular end section of the operating part in question and a fixing connection piece which can be plug-connected to said tubular end section.

8. Functional hose instrument according to any one of claims 1 to 7, further **characterized in that**
- the first operating part has the hose fixing arrangement and the second operating part has the wire fixing arrangement, or
- the first operating part has the wire fixing arrangement and the second operating part has the hose fixing arrangement.

9. Functional hose instrument according to any one of claims 2 to 8, further **characterized in that**
- the operator control rotary element is arranged distally in front of the operator control slide element, or
- the operator control rotary element is arranged proximally behind the operator control slide element.

10. Functional hose instrument according to any one of claims 1 to 9, further **characterized by** an operator control handle (5) comprising a handle body (6) which is designed for releasably receiving the operating unit (7, 8) and has a coupling element (36) for axially fixed coupling of the first operating part.

11. Functional hose instrument according to claim 10, further **characterized in that** the coupling element of the handle body is designed for rotationally movable coupling of the first operating part.

12. Functional hose instrument according to claim 10 or 11, further **characterized in that** the handle body has an axial guide for the operator control slide element.

13. Functional hose instrument according to any one of claims 10 to 12, further **characterized in that** the operator control rotary element is arranged at a distal or proximal end side of the handle body.

14. Functional hose instrument according to any one of claims 10 to 13, further **characterized in that** the handle body is of multi-part design, wherein it comprises a handle base part (6a) and a handle coupling part (6b) which can be releasably connected to said handle base part, and wherein the handle coupling part is designed for releasably receiving the operating unit (7, 8) and has the coupling element for coupling the first operating part.

15. Functional hose instrument according to any one of claims 1 to 14, further **characterized in that** at least one of the two wire guide openings (27, 28) is an opening which is closed at the circumference.

## Revendications

1. Instrument à tuyau souple fonctionnel à commande manuelle, en particulier instrument à tuyau souple fonctionnel endoscopique, comportant
- une partie fonctionnelle (1) en forme tubulaire et une partie fonctionnelle (2) en forme de fil s'étendant dans cette dernière, les deux parties fonctionnelles pouvant être déplacées axialement l'une par rapport à l'autre afin d'exercer une fonction utilitaire à une extrémité distale, et
- une unité de commande adaptée pour être couplée de manière amovible à un corps de poignée de manœuvre et disposée sur une portion d'extrémité proximale desdites parties fonctionnelles et comprenant une première et une deuxième partie de commande (7, 8) couplées l'une à l'autre de façon mobile axialement l'une par rapport à l'autre,
dans lequel
- l'une des deux parties de commande comporte une fixation de tuyau par laquelle la partie fonctionnelle tubulaire est fixée à celle-ci, et l'autre des deux parties de commande comporte une fixation de fil par laquelle la partie fonctionnelle en forme de fil est fixée à celle-ci,
- la première partie de commande (7) comporte un élément de fixation axiale (9) pour fixer axialement la première partie de commande au corps de poignée de manœuvre, et
- la deuxième partie de commande (8) comporte un élément coulissant de manœuvre (10) actionné par le doigt,
**caractérisé en ce que**
- la fixation de fil comprend deux parties de fixation de fil (25, 26) qui peuvent être tournées l'une par rapport à l'autre autour d'un axe longitudinal depuis une position de libération de fil jusque dans une position de serrage de fil et qui présentent chacune une ouverture de passage de fil (27, 28) axiale excentrée, les deux ouvertures de passage étant disposées axialement l'une derrière l'autre et étant alignées dans la position de libération de fil et étant désalignées dans la position de serrage de fil.

2. Instrument à tuyau souple fonctionnel selon la revendication 1,
**caractérisé en outre en ce que**
la première partie de commande (7) comprend un élément rotatif de manœuvre (11) actionné par le doigt.

3. Instrument à tuyau souple fonctionnel selon la revendication 1 ou 2,
**caractérisé en outre en ce que**
la première partie de commande (7) présente une portion conique distale (12) pourvue d'un évidement axial (19) dans lequel la deuxième partie de commande (8) peut être insérée au moins partiellement.

4. Instrument à tuyau souple fonctionnel selon l'une des revendications 1 à 3,
**caractérisé en outre en ce que**
la deuxième partie de commande comporte un corps de fixation (20) couplé à la première partie de commande de manière fixe en rotation et mobile axialement pour fixer la partie fonctionnelle associée, l'élément coulissant de manœuvre étant couplé au corps de fixation de manière mobile en rotation et axialement fixe.

5. Instrument à tuyau souple fonctionnel selon l'une des revendications 1 à 4,
**caractérisé en outre en ce que**
l'une des deux parties de commande comporte une douille de guidage (13) et l'autre partie de commande comporte un élément de guidage (20) qui est guidé de manière fixe en rotation et mobile axialement dans cette dernière.

6. Instrument à tuyau souple fonctionnel selon la revendication 5,
**caractérisé en outre en ce que**
les deux parties de commande comprennent des butées d'extrémité pour limiter de part et d'autre le mouvement axial relatif de la douille de guidage et de l'élément de guidage.

7. Instrument à tuyau souple fonctionnel selon l'une des revendications 1 à 6,
**caractérisé en outre en ce que**
les deux parties de fixation de fil sont formées par une portion d'extrémité tubulaire de la partie de commande respective et par un manchon de fixation qui peut être enfiché sur celle-ci.

8. Instrument à tuyau souple fonctionnel selon l'une des revendications 1 à 7,
**caractérisé en outre en ce que**
- la première partie de commande comprend la fixation de tuyau et la deuxième partie de commande comprend la fixation de fil, ou
- la première partie de commande comprend la fixation de fil et la deuxième partie de commande comprend la fixation de tuyau.

9. Instrument à tuyau souple fonctionnel selon l'une des revendications 2 à 8,
**caractérisé en outre en ce que**
- l'élément rotatif de manœuvre est situé en position distale devant l'élément coulissant de manœuvre, ou
- l'élément rotatif de manœuvre est situé en position proximale derrière l'élément coulissant de manœuvre.

10. Instrument à tuyau souple fonctionnel selon l'une des revendications 1 à 9,
**caractérisé en outre par**
une poignée de manœuvre (5) ayant un corps de poignée (6) qui est conçu pour recevoir de manière amovible l'unité de commande (7, 8) et qui comprend un élément de couplage (36) pour le couplage axialement fixe de la première partie de commande.

11. Instrument à tuyau souple fonctionnel selon la revendication 10,
**caractérisé en outre en ce que**
l'élément de couplage du corps de poignée est conçu pour le couplage mobile en rotation de la première partie de commande.

12. Instrument à tuyau souple fonctionnel selon la revendication 10 ou 11,
**caractérisé en outre en ce que**
le corps de poignée comprend un guidage axial pour l'élément coulissant de manœuvre.

13. Instrument à tuyau souple fonctionnel selon l'une des revendications 10 à 12,
**caractérisé en outre en ce que**
l'élément rotatif de manœuvre est disposé sur une face frontale distale ou proximale du corps de poignée.

14. Instrument à tuyau souple fonctionnel selon l'une des revendications 10 à 13,
**caractérisé en outre en ce que**
le corps de poignée est réalisé en plusieurs parties, comprenant une partie de base de poignée (6a) et une partie de couplage de poignée (6b) pouvant être reliée à celle-ci de façon amovible, et la partie de couplage de poignée est conçue pour recevoir de façon amovible l'unité de commande (7, 8) et comprend l'élément de couplage pour coupler la première partie de commande.

15. Instrument à tuyau souple fonctionnel selon l'une des revendications 1 à 14,
**caractérisé en outre en ce que**
l'une au moins des deux ouvertures de passage de fil (27, 28) est une ouverture fermée du côté circonférentiel.
